(19) **Europäisches Patentamt / European Patent Office / Office européen des brevets**

(11) **EP 2 931 417 B1**

(12) **FASCICULE DE BREVET EUROPEEN**

(45) Date de publication et mention de la délivrance du brevet:
**03.11.2021 Bulletin 2021/44**

(21) Numéro de dépôt: **13802962.4**

(22) Date de dépôt: **10.12.2013**

(51) Int Cl.:
*B01J 20/18* (2006.01)    *B01J 20/28* (2006.01)
*B01J 20/30* (2006.01)    *C07C 29/76* (2006.01)
*C07C 37/82* (2006.01)    *C07C 7/13* (2006.01)

(86) Numéro de dépôt international:
**PCT/EP2013/076021**

(87) Numéro de publication internationale:
**WO 2014/090771 (19.06.2014 Gazette 2014/25)**

(54) **ADSORBANTS ZÉOLITHIQUES ET LEURS UTILISATIONS**

ZEOLITHISCHES ADSORPTIONSMITTEL UND DEREN VERWENDUNGEN

ZEOLITIC ADSORBENTS AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.12.2012 FR 1261964**

(43) Date de publication de la demande:
**21.10.2015 Bulletin 2015/43**

(73) Titulaires:
- **ARKEMA FRANCE**
  **92700 Colombes (FR)**
- **IFP Energies nouvelles**
  **92500 Rueil-Malmaison (FR)**

(72) Inventeurs:
- **BOUVIER, Ludivine**
  **64300 Orthez (FR)**
- **KIEGER, Stéphane**
  **78500 Sartrouville (FR)**
- **LAROCHE, Catherine**
  **69390 Vernaison (FR)**
- **LEFLAIVE, Philibert**
  **69780 Mions (FR)**

(74) Mandataire: **Bandpay & Greuter**
**30, rue Notre-Dame des Victoires**
**75002 Paris (FR)**

(56) Documents cités:
**FR-A1- 2 903 978    FR-A1- 2 925 366
FR-A1- 2 925 367**

- **None**

**Description**

DOMAINE TECHNIQUE

L'invention concerne des adsorbants, sous forme d'agglomérats (adsorbant zéolithique aggloméré) à base de petits cristaux de zéolithe X comprenant du baryum ou du baryum et du potassium, leur procédé de préparation et leurs utilisations.

Ces adsorbants présentent à la fois une capacité d'adsorption optimisée et une résistance mécanique optimisée et peuvent être utilisés plus particulièrement pour la production de para-xylène très pur à partir d'une charge d'hydrocarbures aromatiques contenant des isomères à 8 atomes de carbone.

TECHNIQUE ANTERIEURE

L'utilisation d'adsorbants zéolithiques agglomérés constitués de zéolithes X ou Y comprenant, outre des cations sodium, des ions baryum, potassium ou strontium, seuls ou en mélanges, pour adsorber sélectivement le para-xylène dans un mélange d'hydrocarbures aromatiques, est bien connue de l'art antérieur.

Les brevets US 3 558 730, US 3 558 732, US 3 626 020 et US 3 663 638 montrent que des adsorbants zéolithiques comprenant des aluminosilicates à base de sodium et de baryum (US 3 960 774) ou à base de sodium, de baryum et de potassium, sont efficaces pour la séparation du para-xylène présent dans des coupes aromatiques en C8 (coupes comprenant des hydrocarbures aromatiques à 8 atomes de carbone).

Les adsorbants zéolithiques décrits dans le brevet US 3 878 127 sont utilisés comme agents d'adsorption dans les procédés en phase liquide, de préférence de type contre-courant simulé, similaires à ceux décrits dans le brevet US 2 985 589 et qui s'appliquent, entre autres, aux coupes aromatiques en C8.

Le brevet FR 2925366 décrit des adsorbants zéolitiques à base de cristaux de zéolite LSX utilisés dans des procédés de séparation de sucres, d'alcools polyhydriques, d'isomères de toluène substitué, des crésols ou de récupération de paraxylène. Dans les brevets listés ci-dessus, les adsorbants zéolithiques se présentent sous forme de poudre ou sous forme d'agglomérés constitués majoritairement de zéolithe et jusqu'à 20% en poids de liant inerte.

La synthèse des zéolithes X s'effectue habituellement par nucléation et cristallisation de gels de silico-aluminates. Le plus souvent, les cristaux de zéolithes sont préparés à partir de solutions aqueuses sodiques (par exemple solution aqueuse d'hydroxyde de sodium), et, si on le souhaite, les cations sodium peuvent être remplacés (échangés) en totalité ou en partie par d'autres cations, par exemple baryum ou baryum et potassium. Ces échanges cationiques peuvent être effectués avant et/ou après agglomération de la zéolithe pulvérulente avec le liant d'agglomération, selon des techniques classiques connues de l'homme du métier.

La synthèse des zéolithes X conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérats, ces dites formes pouvant être obtenues par extrusion, pastillage, et autres techniques d'agglomération connues de l'homme du métier. Ces agglomérats ne présentent pas les inconvénients inhérents aux matières pulvérulentes.

Ces agglomérats, qu'ils soient sous forme de plaquettes, de billes, d'extrudés, et autres, sont en général constitués de cristaux de zéolithe(s), qui constituent l'élément actif (au sens de l'adsorption) et d'un liant destiné à assurer la cohésion des cristaux sous forme d'agglomérats et de leur conférer une résistance mécanique suffisante pour résister aux vibrations et aux mouvements auxquels ils sont soumis au cours des opérations de séparations des isomères des coupes aromatiques en C8.

Cependant, les propriétés d'adsorption de ces agglomérats sont évidemment réduites par rapport à la poudre de cristaux, en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est bien maintenant connue de l'homme du métier, par exemple sous la dénomination de « zéolithisation ». Pour effectuer facilement cette opération, on utilise des liants zéolithisables, le plus souvent appartenant à la famille de la kaolinite, et de préférence préalablement calcinés à des températures généralement comprises entre 500°C et 700°C.

Une variante consiste à mouler des grains de kaolin puis à zéolithiser le kaolin ; le principe en est exposé dans « Zeolite Molecular Sieves » de D.W. Breck, John Wiley and Sons, New York, (1973), p. 313 et suivantes. Cette technologie a été appliquée avec succès à l'obtention de grains de zéolithe A ou de zéolithe X, constitués jusqu'à 95% en poids de la zéolithe elle-même et d'un résiduel de liant non transformé (voir par exemple US 3 119 660 où on constate que l'obtention de zéolithe X nécessite l'ajout d'une source de silice dans le milieu réactionnel).

La demande de brevet FR 2 789 914 décrit un procédé de fabrication d'agglomérats à base de zéolithe X, de rapport Si/Al compris entre 1,15 et 1,5, échangés au baryum et éventuellement au potassium, en agglomérant de la poudre de ladite zéolithe X avec un liant, une source de silice et de la carboxyméthylcellulose, puis en zéolithisant le liant par immersion de l'agglomérat dans une liqueur alcaline. Après échange des ions de la zéolithe par des ions baryum (et éventuellement potassium) et activation. Les agglomérats ainsi obtenus présentent, du point de vue de l'adsorption du para-xylène contenu dans les coupes aromatiques en C8, des propriétés améliorées par rapport à des adsorbants préparés à partir de la même quantité de zéolithe X et de liant, mais dont le liant n'est pas zéolithisé.

Plus récemment, le brevet US 7 820 869 décrit un procédé de séparation du para-xylène contenu dans des coupes aromatiques, en utilisant un adsorbant de type « binderless », c'est à dire sans matériau amorphe ou avec une quantité inférieure à 2% en poids de matériau amorphe, à base de zéolithe X, dont la perte au feu est comprise entre 3% et 5%. Ces adsorbants sont obtenus après une étape de zéolithisation du liant et ne contiennent pas, ou seulement en quantité inférieure à 2% en poids, et souvent inférieure à 0,5% en poids, de matériau amorphe ou non zéolithique. Ils présentent une capacité d'adsorption et des propriétés de transferts améliorées pour des procédés de séparation à basse température et à temps de cycle court. En revanche, aucune information n'est donnée sur la résistance mécanique de telles particules « binderless ».

Outre de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (*ibid.,* page 243), que, dans le cas d'un adsorbant sous forme d'agglomérats (adsorbant zéolithique aggloméré), le transfert de matière global dépend de l'addition de la résistance diffusionnelle intra-cristalline et de la résistance diffusionnelle entre les cristaux. La résistance diffusionnelle intra-cristalline est proportionnelle au carré des rayons des cristaux et inversement proportionnelle à la diffusivité des molécules intracristalline.

La résistance diffusionnelle entre les cristaux (également appelée résistance macroporeuse) est quant à elle proportionnelle au carré des rayons des agglomérats et inversement proportionnelle à la diffusivité des molécules dans les macropores. Pour une structure zéolithique donnée, une taille d'agglomérat donnée et une température de fonctionnement donnée, les diffusivités sont fixées, et le seul moyen d'améliorer le transfert de matière consiste à réduire le diamètre des cristaux. Un gain sur le transfert global sera ainsi obtenu en réduisant la taille des cristaux.

Pour estimer cette amélioration de la cinétique de transfert, on peut utiliser la théorie des plateaux décrite par Ruthven dans « Principles of Adsorption and Adsorption Processes », ibid., pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Le brevet US 7 812 208 décrit un procédé de séparation du para-xylène contenu dans des coupes aromatiques, utilisant un adsorbant de type « binderless », c'est à dire sans matériau amorphe ou avec une quantité inférieure à 2% en poids de matériau amorphe, à base de zéolithe X, ayant une taille moyenne de cristaux inférieure à 1,8 $\mu$m. Ces adsorbants sont obtenus après une étape de zéolithisation du liant. Ces adsorbants présentent des propriétés de transfert et d'adsorption améliorées et ne contiennent pas, ou seulement en quantité inférieure à 2% en poids, et souvent inférieure à 0,5% en poids, de matériau amorphe ou non zéolithique. En revanche, aucune information n'est donnée sur la résistance mécanique de telles particules « binderless ».

Une troisième propriété de l'adsorbant nécessaire pour garantir de bonnes performances du procédé de séparation en phase liquide de type contre-courant simulé est d'avoir une bonne résistance mécanique. En effet, en condition standard de fonctionnement de ce type de procédé, une contrainte mécanique importante s'applique sur l'adsorbant au sein des unités industrielles, entraînant la formation de particules fines, qui induisent une détérioration des performances (voir par exemple « Primary Analysis on State of Xylene Adsorption Unit », Li et al., Jingxi Shiyou Huagong, 2004, (4), 54-55), et ce d'autant plus que la résistance mécanique de l'adsorbant sera faible.

Ainsi, la demande de brevet FR 2 903 978 décrit un procédé de fabrication d'agglomérats à base de zéolithe X, à petits cristaux, de taille inférieure à 1,7 $\mu$m, de rapport molaire Si/Al, tel que 1,15 < Si/Al ≤ 1,5, échangés au baryum, et éventuellement au potassium, en agglomérant de la poudre de zéolithe X avec un liant, une source de silice, puis en zéolithisant le liant par immersion des agglomérats dans une liqueur alcaline. Après échange des ions de la zéolithe par des ions baryum (et éventuellement potassium) et activation, les agglomérats ainsi obtenus présentent des propriétés de transfert améliorées dans les procédés de séparation du para-xylène contenu dans les coupes aromatiques tout en renforçant leur résistance mécanique.

Par conséquent, il est connu que les adsorbants zéolithiques agglomérés à base de zéolithe X échangée au baryum ou (baryum et potassium) présentent de bonnes propriétés d'adsorption des xylènes, une bonne sélectivité du para-xylène présent dans les coupes aromatiques en C8, en phase liquide.

Il est par ailleurs connu de WO 2008/009845 que de petits cristaux de zéolithe(s) apportent en général un meilleur transfert de matière, mais une moins bonne résistance mécanique que les cristaux de la même zéolithe de taille supérieure.

L'homme du métier s'attend ainsi à ce que des adsorbants zéolithiques agglomérés, à base de zéolithe X à petits cristaux, comprenant du baryum ou du (baryum et potassium) présentent de bonnes propriétés d'adsorption du para-xylène, une bonne sélectivité et un bon transfert de matière, et s'attend donc à ce que de tels adsorbants présentent de bonnes performances initiales de séparation du para-xylène contenu dans les coupes aromatiques en C8 dans un procédé en phase liquide, par exemple de type contre-courant simulé.

L'art antérieur enseigne que la zéolithisation d'adsorbants agglomérés permet d'augmenter la capacité d'adsorption. D'après l'art antérieur, l'homme de l'art comprend qu'il peut réaliser une conversion totale du liant en zéolithe afin d'obtenir une capacité maximale d'adsorption sans affecter a *priori* les propriétés mécaniques desdits adsorbants agglomérés.

Or les inventeurs ont maintenant découvert que, après conversion totale du liant en zéolithe, les propriétés mécaniques ne sont pas toujours conservées ou optimisées. Il reste par conséquent un besoin pour des adsorbants zéolithiques agglomérés présentant de bonnes propriétés mécaniques, et de bonnes propriétés de sélectivité, notamment au regard de la séparation d'isomères du xylène à partir de coupes aromatiques en C8. Les inventeurs ont ainsi découvert qu'il existe un compromis entre optimum d'adsorption et optimum de résistance mécanique.

La présente invention a ainsi pour objectif de fournir des adsorbants zéolithiques agglomérés, à base de zéolithe X à petits cristaux, comprenant du baryum ou du baryum et du potassium, lesdits adsorbants combinant :

  o une bonne sélectivité,
  o un transfert de matière amélioré,
  o une capacité d'adsorption optimale et
  o une bonne résistance mécanique,

pour la séparation du paraxylène contenu dans les coupes aromatiques en C8 dans un procédé en phase liquide, par exemple de type contre-courant simulé.

Les inventeurs ont constaté que les adsorbants zéolithiques à base de zéolithe X à petits cristaux agglomérés avec un liant zéolithisable, et dont le liant a été soumis à une réaction de zéolithisation totale ou quasi-totale de façon à convertir totalement ou quasi-totalement respectivement le liant en matière active, ne présentent pas une capacité d'adsorption maximale ni une bonne résistance mécanique.

Les inventeurs ont découvert que le meilleur compromis entre capacité d'adsorption maximale et résistance mécanique élevée se situe non pas pour pour un degré de zéolithisation maximal mais pour un degré de zéolithisation telle que la teneur en phase non zéolithique de l'adsorbant est comprise entre 2% et 5% en poids par rapport au poids total dudit adsorbant.

EXPOSE DE L'INVENTION

La présente invention a ainsi pour premier objet un adsorbant zéolithique aggloméré, à propriétés optimisées, notamment pour la séparation du para-xylène des coupes aromatiques en C8. L'adsorbant zéolithique aggloméré présente des propriétés maximales de sélectivité vis-à-vis du para-xylène et de transfert de matière, tout en présentant une résistance mécanique maximale associée à une capacité d'adsorption optimisée et sont particulièrement adaptés pour une utilisation dans un procédé de séparation du para-xylène en phase liquide, de préférence de type contre-courant simulé.

Ainsi, la présente invention concerne un adsorbant zéolithique aggloméré, à base de cristaux de zéolithe X et d'au moins une phase non zéolithique, adsorbant dans lequel :

- les cristaux de zéolithe X présentent :

  i. un diamètre moyen en nombre inférieur ou égal à 1,7 $\mu$m, de préférence inférieur ou égal 1,5 $\mu$m et de préférence encore inférieur ou égal 1,2 $\mu$m,
  ii. un rapport atomique Si/Al compris entre 1,00 et 1,50, de préférence entre 1,05 et 1,50 et de manière encore préférée entre 1,10 et 1,50, bornes incluses,

- la teneur pondérale en phase non zéolithique (PNZ) est telle que 2,0% < PNZ < 5,0%, de préférence telle que 3,0% < PNZ < 5,0%, de préférence encore telle que 3,0% < PNZ < 4,0%, avantageusement telle que 3,2% < PNZ < 3,7% en poids de la masse totale de l'adsorbant,
- la teneur pondérale en oxyde de baryum (BaO) est supérieure à 23%, de préférence supérieure à 32 % et de préférence encore supérieure à 33% par rapport à la masse totale de l'adsorbant,
- la teneur pondérale en oxyde de potassium $K_2O$ est inférieure à 9%, de préférence inférieure à 8% et de préférence encore entre 0% et 2%, avantageusement entre 0% et 1%, bornes incluses, par rapport à la masse totale de l'adsorbant, et

- la teneur pondérale totale en oxydes d'ions alcalins ou alcalino-terreux autres que BaO et $K_2O$ est inférieure à 5% et de préférence est comprise entre 0% et 2% et avantageusement entre 0% et 1%, bornes incluses, par rapport à la masse totale de l'adsorbant.

L'adsorbant zéolithique aggloméré selon la présente invention est un adsorbant à base de cristaux de zéolithe de type faujasite, généralement référencé sous le nom de type X. Par « zéolithe X », on entend les zéolithes dont le ratio atomique Si/Al est compris entre 1,00 et 1,50 bornes incluses, de préférence entre 1,05 et 1,50 bornes incluses et de manière encore plus préférée entre 1,10 et 1,50 bornes incluses.

Parmi les zéolithes X, il est maintenant communément admis de reconnaître deux sous-groupes dénommés zéolithes LSX et zéolithes MSX. Les zéolithes LSX présentent un ratio atomique Si/Al égal à environ 1 et les zéolithes MSX présentent un ratio atomique Si/Al compris entre environ 1,05 et environ 1,15, bornes incluses.

Selon un mode de réalisation préféré de la présente invention, les cristaux de zéolithe X présentent un ratio atomique Si/Al compris entre 1,10 et 1,50, bornes incluses. Selon un autre mode de réalisation préféré, les cristaux de zéolithe X sont des cristaux de zéolithe LSX de ratio atomique Si/Al égal à environ 1. L'invention n'exclut cependant pas que l'adsorbant contienne des mélanges de deux ou plusieurs types de zéolithes X tels qu'ils viennent d'être définis.

Selon encore un autre mode de réalisation préféré de la présente invention, le diamètre moyen en nombre des cristaux de zéolithes X est avantageusement compris entre 0,1 $\mu$m et 1,5 $\mu$m, plus avantageusement entre 0,1 $\mu$m et 1,2 $\mu$m, bornes incluses.

Les adsorbants zéolithiques selon la présente invention comprennent ainsi des cristaux de zéolithe(s) X, et au moins une phase non zéolithique (PNZ), c'est-à-dire une phase non cristalline qui est essentiellement inerte vis-à-vis de l'adsorption. Le taux de cristallinité de l'adsorbant selon l'invention est mesuré par analyse par diffraction de rayons X, connue de l'homme du métier sous l'acronyme DRX.

L'adsorbant zéolithique de l'invention est de préférence sous la forme d'agglomérats, c'est-à-dire qu'il est constitué de cristaux de zéolithe(s) et d'au moins une phase non zéolithique comprenant au moins un liant d'agglomération permettant la cohésion des cristaux entre eux. L'adsorbant zéolithique aggloméré de l'invention est indifféremment dénommé adsorbant zéolithique aggloméré, adsorbant zéolithique ou encore plus simplement agglomérat(s), dans la suite du présent exposé.

Selon encore un autre mode de réalisation de l'invention, l'adsorbant zéolithique présente une teneur pondérale en oxyde de baryum (BaO) comprise entre 33% et 42%, bornes incluses, et typiquement entre 35% et 38%, bornes incluses, par rapport au poids total de l'adsorbant.

Selon un mode de réalisation préféré, l'adsorbant zéolithique selon l'invention présente une perte au feu mesurée à 950 °C selon la norme NF EN 196-2 comprise entre 4,0 et 7,7%, de préférence entre 4,5 et 6,5 % et avantageusement entre 4,8 et 6%, bornes incluses.

Comme indiqué précédemment, l'adsorbant zéolithique selon la présente invention présente de manière surprenante un compromis optimal entre capacité d'adsorption et résistance mécanique. Cette résistance mécanique est mesurée par la méthode Shell série SMS1471-74 adaptée pour des agglomérés de taille inférieure à 1,6 mm et est généralement supérieure ou égale à 1,8 MPa, plus généralement supérieure ou égale à 2 MPa, typiquement supérieure ou égale à 2,1 MPa.

La capacité d'adsorption est quant à elle quantifiée par mesure du volume microporeux de l'adsorbant, volume évalué d'après l'équation de Dubinin-Raduskevitch par adsorption d'azote ($N_2$) à une température de 77K, après dégazage sous vide à 300°C pendant 16 heures. Le volume microporeux des adsorbants zéolithiques de l'invention a ainsi été mesuré comme étant supérieur à 0,250 cm$^3$/g, typiquement dans une plage allant de 0,256 cm$^3$/g à 0,288 cm$^3$/g.

Un procédé de préparation des adsorbants zéolithiques agglomérés tels qu'ils viennent d'être définis, est décrit mais il ne fait pas partie de la présente invention, procédé qui comprend au moins les étapes de :

a) agglomération de cristaux de zéolithe X de diamètre moyen en nombre inférieur ou égal à 1,7 $\mu$m, de préférence inférieur ou égal à 1,5 $\mu$m, et de préférence encore inférieur ou égal à 1,2 $\mu$m, de rapport atomique Si/Al compris entre 1,00 et 1,50, de préférence entre 1,05 et 1,50, et de manière encore plus préférée compris entre 1,10 et 1,50, bornes incluses, avec un liant contenant au moins 80%, de préférence au moins 90%, de préférence encore au moins 95% en poids d'argile zéolithisable et avec une source de silice, puis mise en forme d'agglomérats, et enfin séchage et calcination desdits agglomérats,

b) zéolithisation dudit liant zéolithisable par mise en contact des agglomérats obtenus à l'étape a) avec une solution basique alcaline,

c) échange cationique des agglomérats de l'étape b) par mise en contact avec une solution d'ions baryum, ou d'ions baryum et d'ions potassium,

d) échange cationique éventuel par mise en contact des agglomérats de l'étape c) avec une solution d'ions potassium,

e) puis lavage et séchage des agglomérats ainsi obtenus, et

f) obtention de l'adsorbant zéolithique par activation des agglomérats obtenus à l'étape e).

La taille des cristaux de zéolithe X utilisées à l'étape a) et des cristaux de zéolithe X dans les agglomérats est mesurée par observation au microscope électronique à balayage (MEB). Cette observation MEB permet également de confirmer la présence de phase non zéolithique comprenant par exemple du liant résiduel (non converti lors de l'étape de zéolithisation) ou toute autre phase amorphe dans les agglomérats.

Les cristaux de zéolithe X commerciaux les plus usuels présentent généralement un diamètre supérieur ou égal à 1,8 $\mu$m. Les cristaux mis en oeuvre dans le cadre de la présente invention présentent de préférence un diamètre moyen en nombre inférieur ou égal à 1,7 $\mu$m, de préférence strictement inférieur à 1,5 $\mu$m, et mieux encore inférieur ou égal à 1,2 $\mu$m. Les cristaux dont le diamètre moyen en nombre est strictement inférieur à 1,2 $\mu$m, sont considérés comme des très petits cristaux. Dans le présent document, on emploie l'appellation « diamètre moyen en nombre » ou bien « taille » pour les cristaux de zéolithe et pour les agglomérats. La méthode de mesure de ces grandeurs est explicitée plus loin dans la description.

L'agglomération et la mise en forme (étape a) peuvent être réalisées selon toutes les techniques connues de l'homme de l'art, telles qu'extrusion, compactage, agglomération, et autres. Les proportions de liant d'agglomération (voir définition plus loin) et de zéolithe mises en oeuvre sont typiquement celles de l'art antérieur, c'est-à-dire de 5 parties à 20 parties en poids de liant pour 95 parties à 80 parties en poids de zéolithe. Les agglomérats issus de l'étape a), qu'ils soient sous forme de billes, d'extrudés ou autres, ont en général un diamètre moyen en nombre, ou leur longueur (plus grande dimension lorsqu'ils ne sont pas sphériques), comprise entre 0,4 mm et 2 mm, et en particulier comprise entre 0,4 mm et 0,8 mm et de préférence entre 0,4 mm et 0,65 mm, bornes incluses.

À l'issue de l'étape a) les particules d'agglomérats les plus fines peuvent être éliminées par cyclonage et/ou tamisage et/ou les particules trop grosses par tamisage ou concassage, dans le cas d'extrudés, par exemple.

Le liant d'agglomération mis en œuvre à l'étape a) contient au moins 80 % de préférence, au moins 90%, de préférence encore au moins 95%, plus particulièrement au moins 96%, en poids, d'argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

La poudre de zéolithe X mise en oeuvre à l'étape a) peut être issue de la synthèse de cristaux de zéolithe X comprenant majoritairement, voir exclusivement des cations sodium, par exemple les zéolithes NaX (ou 13X) mais on ne sortirait pas du cadre de l'invention en utilisant une poudre ayant subi un ou plusieurs échanges cationiques, entre la synthèse sous forme NaX et sa mise en oeuvre à l'étape a). Dans ce cas, les étapes c) et d) d'échange cationique deviennent par conséquent non nécessaires.

La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre la poudre de zéolithe X et le liant, des additifs peuvent également être ajoutés, par exemple des additifs destinés à faciliter l'agglomération ou à améliorer le durcissement des agglomérats formés tels que la lignine, l'amidon, la carboxyméthylcellulose, et autres additifs connus de l'homme du métier.

Après le séchage à l'étape a), la calcination est menée à une température en général comprise entre 500°C et 600°C et permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D.W. Breck, John Wiley and Sons, New York, (1973), p. 314-315.

La zéolithisation du liant d'agglomération est pratiquée selon toute méthode maintenant bien connue de l'homme du métier et peut par exemple être réalisée par immersion du produit de l'étape a) dans une solution basique alcaline, en général aqueuse, par exemple une solution aqueuse d'hydroxyde de sodium et/ou d'hydroxyde de potassium.

Les inventeurs ont découvert que la quantité pondérale minimale de phase non zéolithique ne coïncide pas exactement avec les propriétés mécaniques optimales de résistance en lit (REL), typiquement une REL supérieure ou égale à 1,8 MPa, de préférence supérieure ou égale à 2 MPa, pour le type d'adsorbant zéolithique précédemment défini. Aussi, la zéolithisation doit-elle être finement contrôlée de sorte à obtenir le compromis optimal entre quantité pondérale de phase non zéolithique (PNZ) et résistance mécanique, notamment REL. Comme indiqué précédemment, l'adsorbant zéolithique aggloméré de la présente invention présente une quantité pondérale de phase non zéolithique (PNZ) telle que 2,0% < PNZ < 5,0%.

Les paramètres qui peuvent avoir une influence sur le degré de zéolithisation optimal à appliquer pendant la synthèse de l'adsorbant zéolithique selon l'invention sont notamment la concentration de la solution alcaline utilisée, la température à laquelle est conduite la zéolithisation, ainsi que la durée de la réaction de zéolithisation, c'est-à-dire le temps de séjour de l'adsorbant zéolithique dans ladite solution alcaline utilisée.

Ainsi, l'étape de zéolithisation permet d'atteindre un adsorbant zéolithique atteignant le compromis optimal entre capacité d'adsorption et résistance mécanique, notamment REL. Les exemples présentés plus loin à titre d'illustration de la présente invention montrent que ce compromis optimal est obtenu par ajustement de la concentration en solution basique alcaline, de la température et de la durée de zéolithisation.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures, de préférence encore entre environ 2 heures et 6 heures.

L'étape c) d'échange au baryum des cations de la zéolithe s'effectue selon les méthodes classiques connues de l'homme du métier, et le plus souvent par mise en contact des agglomérats issus de l'étape b) (ou de l'étape d) avec un sel de baryum, tel que le chlorure de baryum ($BaCl_2$), en solution aqueuse à une température comprise entre la température ambiante et 100°C, et de préférence comprise entre 80°C et 100°C. Pour obtenir rapidement des teneurs en oxyde de baryum élevées, i.e. de préférence supérieures à 32% en poids par rapport au poids total de l'aggloméré, et de manière préférée allant de 33% à 42% et avantageusement allant de 35% à 38% en poids par rapport au poids total de l'aggloméré, on préfère opérer avec un large excès d'ions baryum par rapport aux cations de la zéolithe que l'on souhaite échanger, typiquement un excès de l'ordre de 10 à 12, avantageusement en procédant par échanges successifs.

L'échange éventuel au potassium (étape d) peut être pratiqué avant et/ou après l'échange au baryum (étape c). Comme indiqué précédemment, il est également possible d'agglomérer à l'étape a) de la poudre de zéolithe X contenant déjà des ions potassium (pré-échange des cations présents dans la zéolithe X de départ, typiquement cations sodium, par des ions potassium avant l'étape a) et s'affranchir (ou non) de l'étape d).

On procède ensuite à un lavage, généralement et de préférence à l'eau, puis d'un séchage de l'agglomérat ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C. Cette étape e) d'activation a pour but de fixer la teneur en eau, ainsi que la perte au feu de l'adsorbant de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

La présente invention concerne également les utilisations des adsorbants zéolithiques décrits ci-dessus comme agents d'adsorption susceptibles de remplacer avantageusement les agents d'adsorption décrits dans la littérature, à base de zéolithe X, comprenant de l'oxyde de baryum, ou à base de zéolithe X comprenant de l'oxyde de baryum et de l'oxyde de potassium, et notamment dans les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- la séparation des alcools polyhydriques, tels que les sucres.

L'invention concerne notamment un perfectionnement de procédé de récupération de para-xylène à partir de coupes d'isomères aromatiques à 8 atomes de carbone consistant à utiliser, comme agent d'adsorption du para-xylène, un adsorbant zéolithique aggloméré selon l'invention, mis en œuvre dans des procédés en phase liquide mais aussi en phase gazeuse, ledit procédé étant de préférence conduit en présence d'un désorbant.

On peut ainsi séparer le produit désiré (para-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

- nombre de lits 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (stand alone) et entre 0,7 et 1,4 pour une unité d'adsorption combinée à une unité de cristallisation,
- taux de recyclage compris entre 2,5 et 12, de préférence entre 3,5 et 6.

On pourra sur ce sujet se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le para-diéthylbenzène (PDEB). La sélectivité des adsorbants selon l'invention pour l'adsorption du para-xylène contenu dans des coupes aromatiques en C8 est optimale lorsque leur perte au feu mesurée à 900°C est comprise en général entre 4,0% et 7,7%, et de préférence entre 4,7% et 6,7%.

DESCRIPTION DES FIGURES

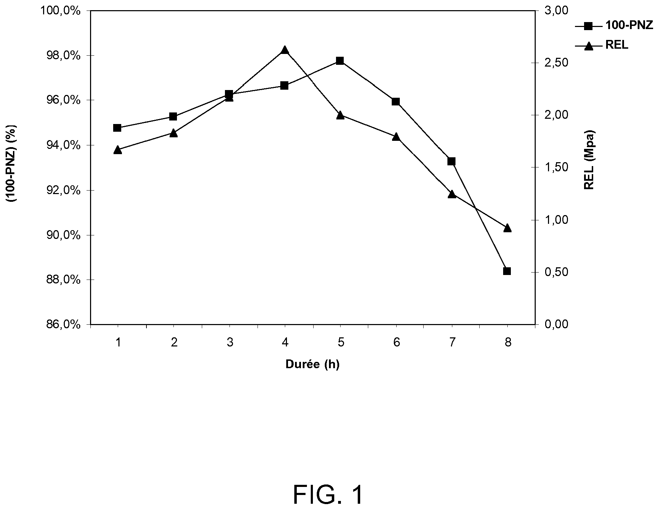

La Figure 1 présente l'évolution de la résistance mécanique en lit (REL) et du taux de phase non-zéolithique (PNZ), en fonction d'un des facteurs permettant le contrôle de la zéolithisation qui est ici la durée de la réaction de zéolithisation. De manière inattendue, cette figure montre que prolonger le temps de zéolithisation, dans le but de diminuer le taux de phase non-zéolithique, entraîne au contraire une diminution drastique de la phase cristalline et en même temps de la résistance mécanique en lit de l'adsorbant zéolithique. La réaction de zéolithisation doit donc être finement contrôlée pour obtenir un adsorbant zéolithique présentant à la fois une capacité optimale en termes d'adsorption et des propriétés mécaniques optimales en termes de résistance mécanique en lit.

TECHNIQUES DE CARACTERISATION

**Granulométrie des cristaux** :

L'estimation du diamètre moyen en nombre des cristaux de zéolithe X utilisées à l'étape a) et des cristaux de zéolithe X contenue dans les agglomérés est réalisée par observation au microscope électronique à balayage (MEB).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié, par exemple le logiciel Smile View de l'éditeur LoGraMi. La précision est de l'ordre de 3%.

**Analyse chimique des adsorbants zéolithiques** - **rapport Si/Al et taux d'échange** :

Une analyse chimique élémentaire du produit final obtenu à l'issue des étapes a) à f) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe au sein de l'agglomérat, et de vérifier la qualité de l'échange ionique décrit à l'étape c) et à l'étape optionnelle d). Dans la description de la présente invention, l'incertitude de mesure du ratio atomique Si/Al est de $\pm$ 5%.

La qualité de l'échange ionique est liée au nombre de mole d'oxyde de sodium, $Na_2O$, restant dans l'adsorbant zéolithique aggloméré après échange. Plus précisément, le taux d'échange par les ions baryum est estimé en évaluant le rapport entre le nombre de moles d'oxyde de baryum, BaO, et le nombre de moles de l'ensemble (BaO + $Na_2O$). De même, le taux d'échange par les ions baryum et potassium est estimé en évaluant le rapport entre le nombre de moles de l'ensemble oxyde de baryum + oxyde de potassium (BaO + $K_2O$) et le nombre de moles de l'ensemble (BaO + $K_2O$ + $Na_2O$). Il est à noter que les teneurs en différents oxydes sont données, en pourcentage en poids par rapport au poids total de l'adsorbant zéolithique anhydre.

**Granulométrie des adsorbants zéolithiques** :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les adsorbants zéolithiques agglomérés. La précision est de l'ordre de 0,01 mm pour la gamme de taille d'agglomérats de l'invention.

**Résistance mécanique des adsorbants zéolithiques** :

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm mais doit être adapté selon la granulométrie des agglomérés que l'on cherche à caractériser.

Les agglomérats de la présente invention, généralement sous forme de billes ou d'extrudés, ont en général un diamètre moyen en nombre ou une longueur, i.e. plus grande dimension dans le cas des agglomérés non sphériques, comprise entre 0,4 mm et 2 mm, et en particulier comprise entre 0,4 mm et 0,8 mm et de préférence entre 0,4 mm et 0,65 mm. Par conséquent, un tamis de 200 $\mu$m est utilisé à la place du tamis de 425 $\mu$m mentionné dans la méthode Shell standard SMS1471-74.

Le protocole de mesure est le suivant : un échantillon de 20 cm$^3$ d'agglomérats, préalablement tamisé avec le tamis adapté (200 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérats (utilisation de billes de 2 mm de diamètre pour des particules de forme sphérique de diamètre strictement inférieur à 1,6 mm). Les fines obtenues aux différents paliers de pression sont séparées par tamisage (tamis adapté de 200 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant zéolithique et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

**Phase non zéolithique des adsorbants zéolithiques** :

Le taux de phase non zéolithique, par exemple liant résiduel non zéolithisé ou toute autre phase amorphe, après zéolithisation est calculé selon l'équation suivante :

$$PNZ = 100 - \Sigma\ (PZ),$$

où PZ représente la somme des quantités des fractions zéolithiques X au sens de l'invention.

La quantité des fractions zéolithiques X est mesurée par analyse par diffraction de rayons X, connue de l'homme du métier sous l'acronyme DRX. Cette analyse est réalisée sur un appareil de la marque Bruker, puis la quantité des fractions zéolithiques X est évaluée au moyen du logiciel TOPAS de la société Bruker.

**Volume microporeux** :

La cristallinité des agglomérats est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction. Préalablement à l'adsorption, l'adsorbant zéolithique est dégazé entre 300°C et 450°C pendant une durée de 9 heures à 16 heures, sous vide (P < 6,7.10$^{-4}$ Pa). La mesure de l'isotherme d'adsorption d'azote à 77K est ensuite effectuée sur un appareil de type ASAP 2010 M de Micromeritics, en prenant au moins 35 points de mesure à des pressions relatives de rapport $P/P_0$ compris entre 0,002 et 1. Le volume microporeux est déterminé selon Dubinin et Raduskevitch à partir de l'isotherme obtenue, en appliquant la norme ISO 15901-3:2007. Le volume microporeux évalué selon Dubinin et Raduskevitch s'exprime en cm$^3$ d'adsorbat liquide par gramme d'adsorbant. L'incertitude de mesure est de $\pm$ 0,003 g/cm$^3$.

**Perte au feu des adsorbants zéolithiques** :

La perte au feu est déterminée en atmosphère oxydante, par calcination de l'échantillon à l'air à une température de 950°C $\pm$ 25°C, comme décrit dans la norme NF EN 196-2 (avril 2006). L'écart type de mesure est inferieur à 0,1%.

**Caractérisation de l'adsorption en phase liquide par perçage** :

La technique utilisée pour caractériser l'adsorption de molécules en phase liquide sur un solide poreux est la technique dite de perçage, décrite par Ruthven dans « Principles of Adsorption and Adsorption Processes » (Chapitres 8 et 9, John Wiley & Sons, 1984) qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

EXEMPLES

**Exemple A :** Synthèse de cristaux de zéolithe X, de rapport atomique Si/Al = 1,25, de diamètre moyen en nombre de 1,0 $\mu$m et de rapport atomique Na/Al = 1

On prépare un gel de composition molaire 3,5 Na$_2$O - 2,8 SiO$_2$ - Al$_2$O$_3$ - 130 H$_2$O par mélange des réactifs suivants : silicate de sodium, aluminate de sodium et eau. Le gel est mûri à 35°C pendant 20 heures, et on opère une cristallisation pendant 4 heures à 100°C.

Les cristaux obtenus après filtration et lavage sont identifiés par diffraction des rayons X (analyse DRX) comme étant des cristaux de faujasite. L'analyse chimique du solide donne un rapport atomique Si/Al = 1,25. Le volume microporeux évalué d'après l'équation de Dubinin-Raduskevitch tel que décrit dans la partie technique de caractérisation et exprimé en cm$^3$ par gramme d'adsorbant sec est de 0,345 $\pm$ 0,003 cm$^3$/g. L'analyse de la taille des cristaux de zéolithe est réalisée par microscopie électronique à balayage et montre que leur diamètre moyen en nombre est de 1,0 $\mu$m.

**Exemple B** : Synthèse de cristaux de zéolithe X, Si/Al = 1,20, diamètre 0,8 $\mu$m

On prépare un gel de composition molaire 4 Na$_2$0 - 2,8 SiO$_2$ - Al$_2$O$_3$ - 130 H$_2$O par mélange des réactifs : silicate de soude et aluminate de sodium et eau, au moyen d'une turbine, on laisse le gel mûrir à 35 °C pendant 20 heures et on opère une cristallisation de 4 heures à 100 °C.

Les cristaux obtenus après filtration et lavage sont identifiés par diffraction des rayons X (analyse DRX) comme étant des cristaux de Faujasite. L'analyse chimique du solide donne un rapport atomique Si/Al = 1,20 $\pm$ 0,03. Le volume microporeux évalué d'après l'équation de Dubinin- Raduskevitch, comme décrit précédemment, et exprimé en cm$^3$/g d'adsorbant sec, est de 0,344 $\pm$ 0,003 cm$^3$/g. L'analyse de la taille des cristaux de zéolithe est réalisée par microscopie électronique à balayage : leur diamètre moyen en nombre est de 0,8 $\mu$m.

**Préparation des adsorbants zéolithiques**

On prépare un mélange homogène et on agglomère 800 g de cristaux de zéolithe NaX préparée selon les modes opératoires décrit dans les exemples A ou B avec 105 g de kaolin (exprimés en équivalent calciné) et 45 g de silice colloïdale vendue sous la dénomination commerciale Klebosol$^®$30 (contenant 30% en poids de SiO$_2$ et 0,5% de Na$_2$O) avec la quantité d'eau qui permet l'extrusion du mélange. Les extrudés sont séchés, concassés de manière à récupérer des grains dont le diamètre moyen en nombre est égal à 0,7 mm, puis calcinés à 550°C sous courant d'azote pendant 2 heures.

**Exemple 1**

200 g d'agglomérats obtenus à partir de la poudre synthétisée dans l'exemple A sont placés dans un réacteur en verre muni d'une double enveloppe régulée à une température de 100°C $\pm$ 1°C, puis on ajoute 1,5 L d'une solution aqueuse d'hydroxyde de sodium de concentration 2,5 M et on laisse le milieu réactionnel sous agitation pendant une durée variable de 1 heure à 8 heures comme indiqué dans le tableau ci-après.

On procède ensuite au lavage des agglomérats en 3 opérations successives de lavage à l'eau suivi de la vidange du réacteur. On s'assure de l'efficacité du lavage en mesurant le pH final des eaux de lavage compris entre 10,0 et 10,5.

Ces agglomérats sont engagés dans une réaction d'échange cationique par action d'une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.

Les différents produits sont caractérisés afin de déterminer par toutes les techniques analytiques décrites ci-dessus : la teneur en phase non zéolithique (PNZ), le volume microporeux selon Dubinin-Raduskevitch (Vol DR) et la résistance mécanique en lit (REL). Les résultats sont donnés dans le tableau 1.

La perte au feu mesurée, comme décrit précédemment, est de 5,4% $\pm$ 0,1% pour chaque échantillon. Les taux d'échange baryum des agglomérés calculés à partir des analyses élémentaires des oxydes de baryum et de sodium par WDXRF comme décrit dans les techniques de caractérisation est de 99,7 $\pm$ 0,2%.

--**Tableau 1** --

| Durée (h) | REL (MPa) | Vol DR (cm$^3$/g) | PNZ |
|-----------|-----------|-------------------|-------|
| 1 | 1,67 | 0,250 | 6,4% |
| 2 | 1,83 | 0,254 | 4,9% |
| 3 | 2,17 | 0,257 | 3,7% |
| 4 | 2,62 | 0,258 | 3,4% |
| 5 | 2,00 | 0,261 | 2,2% |
| 6 | 1,80 | 0,256 | 4,0% |
| 7 | 1,25 | 0,249 | 6,7% |
| 8 | 0,93 | 0,236 | 11,6% |

Les valeurs du tableau 1 sont reportées sous forme de graphique dans la Figure 1. On constate qu'il n'est pas possible d'obtenir des adsorbants zéolithiques agglomérés qui combinent à la fois une capacité d'adsorption maximale et une résistance mécanique maximale. Un compromis capacité d'adsorption optimale / résistance mécanique optimale est alors obtenu pour une teneur en phase non zéolithique (PNZ) comprise entre 2 et 5% en poids par rapport au poids de l'aggloméré et mesuré par DRX.

**Exemple 2** : Essais avec différentes concentrations d'hydroxyde de sodium

200 g d'agglomérats obtenus à partir de la poudre synthétisée dans l'exemple A sont placés dans un réacteur en verre muni d'une double enveloppe régulée à une température de 100°C $\pm$ 1°C puis on ajoute 1,5 L d'une solution aqueuse d'hydroxyde de sodium de concentration allant de 0,5 M à 5,5 M, comme indiqué dans le tableau 2, et on laisse le milieu réactionnel sous agitation pendant une durée de 4 à 5 heures.

On procède ensuite au lavage des agglomérats en 3 opérations successives de lavage à l'eau suivi de la vidange du réacteur. On s'assure de l'efficacité du lavage en mesurant le pH final des eaux de lavage qui est compris entre 10,0 et 10,5.

Ces agglomérats sont échangés, comme décrit à l'exemple 1, au moyen d'une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.

Les différents produits sont caractérisés afin de déterminer par toutes les techniques analytiques décrites ci-dessus : la perte au feu, la teneur en phase non zéolithique, le volume microporeux et la résistance mécanique. Les résultats sont donnés dans le tableau 2 ci-dessous.

Les taux d'échange baryum des agglomérats calculés à partir des analyses élémentaires des oxydes de baryum et de sodium par WDXRF comme décrit dans les techniques de caractérisation est de 99,6 $\pm$ 0,2%. La perte au feu mesurée comme décrit précédemment est de 5,3% $\pm$ 0,1% pour chaque échantillon.

-- Tableau 2 --

| Concentration (M) | REL (MPa) | Vol DR (cm$^3$/g) | PNZ |
|---|---|---|---|
| 0,5 | 1,20 | 0,242 | 9,7% |
| 2,5 | 2,62 | 0,258 | 3,4% |
| 5,5 | 0,80 | 0,230 | 14,2% |

On constate qu'il n'est pas possible d'obtenir des adsorbants zéolithiques agglomérés qui combinent à la fois une capacité d'adsorption maximale et une résistance mécanique maximale. Un compromis capacité d'adsorption optimale / résistance mécanique optimale est alors obtenu pour une teneur en phase non zéolithique de l'ordre de 3% en poids par rapport au poids de l'aggloméré et mesuré par DRX selon la méthode décrite précédemment.

**Exemple** 3 : Agglomérats à base de cristaux de NaX de 0,8 $\mu$m

200 g d'agglomérats obtenus à partir de la poudre synthétisée dans l'exemple B sont placés dans un réacteur en verre muni d'une double enveloppe régulée à une température de 100°C $\pm$ 1 °C puis on ajoute 1,5 L d'une solution aqueuse d'hydroxyde de sodium de concentration 100 g/L et on laisse le milieu réactionnel sous agitation pendant une durée variable de 1 heure à 8 heures comme indiqué dans le tableau ci-après.
On procède ensuite au lavage des agglomérats en 3 opérations successives de lavage à l'eau suivi de la vidange du réacteur. On s'assure de l'efficacité du lavage en mesurant le pH final des eaux de lavage qui est compris entre 10,0 et 10,5.
Ces agglomérats sont échangés, comme décrit à l'exemple 1, au moyen d'une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.
Les différents produits sont caractérisés afin de déterminer par toutes les techniques analytiques décrites ci-dessus : la perte au feu, la teneur en phase non zéolithique, le volume microporeux et la résistance mécanique. Les résultats sont donnés dans le tableau 3 ci-dessous.
Les taux d'échange baryum des agglomérats calculés à partir des analyses élémentaires des oxydes de baryum et de sodium par WDXRF comme décrit dans les techniques de caractérisation est de 99,7 $\pm$ 0,2%. La perte au feu mesurée comme décrit précédemment est de 5,3% $\pm$ 0,1% pour chaque échantillon.

-- Tableau 3 --

| Durée (h) | REL (MPa) | Vol DR (cm$^3$/g) | PNZ (DRX) |
|---|---|---|---|
| 1 | 1,50 | 0,248 | 6,8% |
| 4 | 2,20 | 0,260 | 2,3% |
| 8 | 0,80 | 0,245 | 7,9% |

**Exemple 4** : Agglomérats à base de cristaux de NaMSX

200 g d'agglomérats obtenus à partir de poudre de NaMSX (K. Schumann et coll., Microporous and Mesoporous Materials, 154, (2012), 119-123) sont placés dans un réacteur en verre muni d'une double enveloppe régulée à une température de 100 $\pm$ 1°C, puis on ajoute 1,5 L d'une solution aqueuse d'hydroxyde de sodium de concentration 100 g/L et on laisse le milieu réactionnel sous agitation pendant une durée variable de 1 à 8 heures comme indiqué dans le tableau 4 ci-après.
On procède ensuite au lavage des agglomérats en 3 opérations successives de lavage à l'eau suivi de la vidange du réacteur. On s'assure de l'efficacité du lavage en mesurant le pH final des eaux de lavage qui est compris entre 10 et 10,5.
Ces agglomérats sont échangés, comme décrit à l'exemple 1, au moyen d'une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 h et enfin activés à 250°C pendant 2 heures sous courant d'azote.

Le différents produits sont caractérisés afin de déterminer par toutes les techniques analytiques décrites ci-dessus : la perte au feu, la teneur en phase non zéolithique, le volume microporeux et la résistance mécanique. Les résultats sont donnés dans le tableau 4 ci-dessous.

Les taux d'échange baryum des agglomérats calculés à partir des analyses élémentaires des oxydes de baryum et de sodium par WDXRF comme décrit dans les techniques de caractérisation est de 99,8 ± 0,2%. La perte au feu mesurée comme décrit précédemment est de 5,4% ± 0,1 % pour chaque échantillon.

-- **Tableau 4** --

| Durée (h) | REL (MPa) | Vol DR (cm$^3$/g) | PNZ |
|-----------|-----------|-------------------|------|
| 1 | 1,90 | 0,242 | 7,3% |
| 4 | 2,90 | 0,254 | 2,7% |
| 8 | 1,20 | 0,238 | 8,8% |

**Exemple 5** : Test en perçage

Un test de perçage (chromatographie frontale) est ensuite réalisé sur les adsorbants zéolithiques agglomérés décrits dans les exemples 1 et 3 pour évaluer leur efficacité. La quantité d'adsorbant utilisée pour ces tests est d'environ 74 g.

Le mode opératoire pour obtenir les courbes de perçage est le suivant :

- Remplissage de la colonne par le tamis et mise en place dans le banc de test.
- Remplissage par le solvant (paradiéthylbenzène à température ambiante.
- Montée progressive à la température d'adsorption sous flux de solvant (5 cm$^3$/min).
- Injection de solvant à 10 cm$^3$/min lorsque la température d'adsorption est atteinte
- Permutation solvant/charge pour injecter la charge (10 cm$^3$/min).
- L'injection de la charge est ensuite maintenue un temps suffisant pour atteindre l'équilibre thermodynamique.
- Collecte et analyse de l'effluent du perçage.

La pression est suffisante pour que la charge reste en phase liquide, soit 1 MPa. La température d'adsorption est de 175°C.

La composition de la charge est la suivante :

- Paraxylène : 45% poids
- Métaxylène : 45% poids
- *Iso*-octane : 10% poids (celui-ci est utilisé comme traceur pour l'estimation des volumes non-sélectifs et n'intervient pas dans la séparation)

Les résultats issus de l'analyse des courbes de perçage telle que décrit dans les techniques de caractérisation sont consignés dans le tableau 5 ci-dessous et sont comparés avec les résultats obtenus avec l'adsorbant selon l'art antérieur (exemple 4 du brevet FR2903978) à partir de test de perçage réalisé dans les mêmes conditions que décrits précédemment.

-- **Tableau 5** --

| Nature du solide | Vol DR (cm$^3$/g) | PNZ | PAF[1] à 900°C | Capacité[2] | Sélectivité[3] $\alpha_{PX/MX}$ | Hauteur de plateau théorique (cm) |
|------------------|-------------------|------|----------------|-------------|-------------------------------|-----------------------------------|
| Aggloméré à base de cristaux de BaX de 1,6 µm (ex 4 FR2903978) | 0,256 | 5,5% | 6,0% | 0,194 | 3,33 | 2,26 |
| Aggloméré à base de cristaux de BaX de 1,0 µm (ex 1) | 0,258 | 3,4% | 5,4% | 0,214 | 3,67 | 2,17 |

(suite)

| Nature du solide | Vol DR (cm³/g) | PNZ | PAF[1] à 900°C | Capacité[2] | Sélectivité[3] $\alpha_{PX/MX}$ | Hauteur de plateau théorique (cm) |
|---|---|---|---|---|---|---|
| Agglomérat à base de cristaux de BaX de 0,8 µm (ex 3) | 0,260 | 2,3% | 5,3% | 0,217 | 3,71 | 1,98 |

(1) PAF : Perte au feu (2) La capacité est exprimée en cm³ d'hydrocarbures aromatiques en C8 adsorbé par gramme d'adsorbant tel quel (3) PX : para-xylène ; MX : méta-xylène

On constate que les adsorbants zéolithiques agglomérés selon l'invention associent simultanément une capacité, une sélectivité et un transfert de matière améliorés par rapport aux adsorbants de l'art antérieur, notamment ceux décrits dans FR2903978.

**Revendications**

1. Adsorbant zéolithique aggloméré, à base de cristaux de zéolithe X et d'au moins une phase non zéolithique, adsorbant dans lequel :

   - les cristaux de zéolithe X présentent :

      i. un diamètre moyen en nombre inférieur ou égal à 1,7 µm, de préférence inférieur ou égal 1,5 µm et de préférence encore inférieur ou égal 1,2 µm,
      ii. un rapport atomique Si/Al compris entre 1,00 et 1,50, de préférence entre 1,05 et 1,50 et de manière encore préférée entre 1,10 et 1,50, bornes incluses,

   - la teneur pondérale en phase non-zéolitique (PNZ) est telle que 2,0% < PNZ < 5,0%, de préférence telle que 3,0% < PNZ < 5,0%, de préférence encore telle que 3,0% < PNZ < 4,0%, avantageusement telle que 3,2% < PNZ < 3,7% en poids de la masse totale de l'adsorbant,
   - la teneur pondérale en oxyde de baryum (BaO) est supérieure à 23%, de préférence supérieure à 32 % et de préférence encore supérieure à 33% en poids par rapport à la masse totale de l'adsorbant,
   - la teneur pondérale en oxyde de potassium $K_2O$ est inférieure à 9%, de préférence inférieure à 8% et de préférence encore entre 0% et 2% et avantageusement entre 0% et 1%, bornes incluses, par rapport à la masse totale de l'adsorbant, et
   - la teneur pondérale totale en oxydes d'ions alcalins ou alcalino-terreux autres que BaO et $K_2O$ est inférieure à 5% et de préférence comprise entre 0% et 2% et avantageusement entre 0% et 1%, bornes incluses, par rapport à la masse totale de l'adsorbant.

2. Adsorbant selon la revendication 1, dans lequel le diamètre moyen en nombre des cristaux de zéolithes X est avantageusement compris entre 0,1 µm et 1,5 µm, plus avantageusement entre 0,1 µm et 1,2 µm, bornes incluses.

3. Adsorbant selon la revendication 1 ou la revendication 2, dans lequel la teneur en oxyde de baryum (BaO) est comprise entre 33% et 42%, bornes incluses, et typiquement entre 35% et 38%, bornes incluses, en poids par rapport au poids total de l'adsorbant.

4. Adsorbant selon l'une quelconque des revendications précédentes, dont la perte au feu mesurée à 950 °C selon la norme NF EN 196-2 est comprise au sens large entre 4,0 et 7,7% et de préférence entre 4,5 et 6,5 % et avantageusement entre 4,8 et 6%, bornes incluses.

5. Adsorbant selon l'une quelconque des revendications précédentes, sous forme d'agglomérats dont le diamètre moyen en nombre est compris entre 0,4 mm et 2 mm, en particulier entre 0,4 mm et 0, 8 mm et de préférence entre 0,4 mm et 0,65 mm, bornes incluses.

6. Utilisation d'un adsorbant selon l'une quelconque des revendications 1 à 5, dans les procédés de :

• séparation de coupes d'isomères aromatiques en C8 et notamment des xylènes,
• séparation d'isomères de toluène substitué tels que nitrotoluène, diéthyltoluène, toluènediamine, et autres,
• séparation des crésols,
• séparation des alcools polyhydriques.

7. Utilisation selon la revendication 6, pour la séparation de para-xylène à partir de coupes d'isomères aromatiques à 8 atomes de carbone.

8. Procédé de récupération de para-xylène à partir de coupes d'isomères d'hydrocarbures aromatiques contenant 8 atomes de carbone, en phase liquide ou en phase gazeuse, par adsorption du para-xylène au moyen d'un adsorbant selon l'une quelconque des revendications 1 à 5, en présence d'un désorbant.

9. Procédé de récupération de paraxylène selon la revendication 8 de type lit mobile simulé, c'est-à-dire à contre courant simulé ou à co-courant simulé.

10. Procédé de récupération de para-xylène selon l'une quelconque des revendications 8 et 9, dans lequel le désorbant est choisi parmi le toluène et le *para*diéthylbenzène.

**Patentansprüche**

1. Agglomeriertes zeolithisches Adsorptionsmittel auf der Basis von Zeolith-X-Kristallen und von mindestens einer nicht-zeolithischen Phase, wobei bei dem Adsorptionsmittel:

   - die Zeolith-X-Kristalle aufweisen

     i. einen zahlenmittleren Durchmesser von unter oder gleich 1,7 $\mu$m, vorzugsweise von unter oder gleich 1,5 $\mu$m und noch vorzugsweiser von unter oder gleich 1,2 $\mu$m,
     ii. ein Atomverhältnis Si/Al, das zwischen 1,00 und 1,50, vorzugsweise zwischen 1,05 und 1,50 und in noch bevorzugter Weise zwischen 1,10 und 1,50, Grenzwerte eingeschlossen, liegt,

   - der Gewichtsanteil an nicht-zeolithischer Phase (PNZ) derart ist, dass 2,0 % < PNZ < 5,0 %, vorzugsweise derart ist, dass 3,0 % < PNZ < 5,0 %, noch vorzugsweiser derart ist, dass 3,0 % < PNZ < 4,0 %, in vorteilhafter Weise derart ist, dass 3,2 % < PNZ < 3,7 Gew.-% der Gesamtmasse des Adsorptionsmittels ist,
   - der Gewichtsanteil an Bariumoxid (BaO) größer als 23 %, vorzugsweise größer als 32 % und noch vorzugsweiser größer als 33 Gew.-% im Verhältnis zur Gesamtmasse des Adsorptionsmittels ist,
   - der Gewichtsanteil an Kaliumoxid $K_2O$ kleiner als 9 %, vorzugsweise kleiner als 8 % und noch vorzugsweiser zwischen 0 % und 2 % und in vorteilhafter Weise zwischen 0 % und 1 %, Grenzwerte eingeschlossen, im Verhältnis zur Gesamtmasse des Adsorptionsmittels ist, und
   - der Gewichtsanteil insgesamt an Oxiden alkalischer oder erdalkalischer Ionen, die andere als BaO und $K_2O$ sind, kleiner als 5 % ist und vorzugsweise zwischen 0 % und 2 % und in vorteilhafter Weise zwischen 0 % und 1 %, Grenzwerte eingeschlossen, im Verhältnis zur Gesamtmasse des Adsorptionsmittels liegt.

2. Adsorptionsmittel nach Anspruch 1, wobei der zahlenmittlere Durchmesser der Zeolith-X-Kristalle in vorteilhafter Weise zwischen 0,1 $\mu$m und 1,5 $\mu$m, in vorteilhafterer Weise zwischen 0,1 $\mu$m und 1,2 $\mu$m, Grenzwerte eingeschlossen, liegt.

3. Adsorptionsmittel nach Anspruch 1 oder Anspruch 2, wobei der Gehalt an Bariumoxid (BaO) zwischen 33 % und 42 %, Grenzwerte eingeschlossen, und in typischer Weise zwischen 35 % und 38 Gew.-%, Grenzwerte eingeschlossen, im Verhältnis zum Gesamtgewicht des Adsorptionsmittels liegt.

4. Adsorptionsmittel nach einem der vorangehenden Ansprüche, dessen Glühverlust, gemessen bei 950 °C gemäß der Norm NF EN 196-2, im weiten Sinne zwischen 4,0 und 7,7 % und vorzugsweise zwischen 4,5 und 6,5 % und in vorteilhafter Weise zwischen 4,8 und 6 %, Grenzwerte eingeschlossen, liegt.

5. Adsorptionsmittel nach einem der vorangehenden Ansprüche in Form von Agglomeraten, deren zahlenmittlerer Durchmesser zwischen 0,4 mm und 2 mm, insbesondere zwischen 0,4 mm und 0,8 mm und vorzugsweise zwischen 0,4 mm und 0,65 mm, Grenzwerte eingeschlossen, liegt.

6. Verwendung eines Adsorptionsmittels nach einem der Ansprüche 1 bis 5 in den Verfahren:

   • Separieren von Schnitten aromatischer C8-Isomere und vor allem der Xylene,
   • Separieren von Isomeren substituierten Tuolens wie Nitrotoluen, Diethyltoluen, Toluendiamin und anderen,
   • Separieren der Cresole,
   • Separieren der mehrwertigen Alkohole.

7. Verwendung nach Anspruch 6 für die Separation von para-Xylen ausgehend von Schnitten aromatischer Isomere mit 8 Kohlenstoffatomen.

8. Verfahren zur Rückgewinnung von para-Xylen ausgehend von Schnitten von Isomeren aromatischer Kohlenwasserstoffe mit 8 Kohlenstoffatomen in flüssiger Phase oder in gasförmiger Phase durch Adsorption des para-Xylens mittels eines Adsorptionsmittels nach einem der Ansprüche 1 bis 5 in Anwesenheit eines Desorptionsmittels.

9. Verfahren zur Rückgewinnung von Paraxylen nach Anspruch 8 vom Typ simuliertes bewegliches Bett, das heißt, mit simuliertem Gegenstrom oder mit simuliertem Gleichstrom.

10. Verfahren zur Rückgewinnung von para-Xylen nach einem der Ansprüche 8 und 9, wobei das Desorptionsmittel aus dem Toluen und dem para-Diethylbenzol ausgewählt ist.

**Claims**

1. Agglomerated zeolitic adsorbent, based on crystals of zeolite X and on at least one non-zeolitic phase, in which adsorbent:

   - the crystals of zeolite X exhibit:

     i. a number-average diameter of less than or equal to 1.7 $\mu$m, preferably of less than or equal to 1.5 $\mu$m and more preferably of less than or equal to 1.2 $\mu$m,
     ii. an Si/Al atomic ratio of between 1.00 and 1.50, preferably between 1.05 and 1.50 and more preferably between 1.10 and 1.50, limits included,

   - the content by weight of non-zeolitic phase (NZP) is such that 2.0% < NZP < 5.0%, preferably such that 3.0% < NZP < 5.0%, more preferably such that 3.0% < NZP < 4.0%, advantageously such that 3.2% < NZP < 3.7%, by weight of the total mass of the adsorbent,
   - the content by weight of barium oxide (BaO) is greater than 23%, preferably greater than 32% and more preferably greater than 33% by weight, with respect to the total mass of the adsorbent,
   - the content by weight of potassium oxide $K_2O$ is less than 9%, preferably less than 8% and more preferably between 0% and 2% and advantageously between 0% and 1%, limits included, with respect to the total mass of the adsorbent, and
   - the total content by weight of oxides of alkali metal or alkaline earth metal ions, other than BaO and $K_2O$, is less than 5% and preferably comprised between 0% and 2% and advantageously between 0% and 1%, limits included, with respect to the total mass of the adsorbent.

2. Adsorbent according to claim 1, wherein the number-average diameter of the crystals of zeolites X is advantageously between 0.1 $\mu$m and 1.5 $\mu$m, more advantageously between 0.1 $\mu$m and 1.2 $\mu$m, limits included.

3. Adsorbent according to claim 1 or claim 2, wherein the content of barium oxide (BaO) is comprised between 33% and 42%, limits included, and typically between 35% and 38%, limits included, by weight, with respect to the total weight of the adsorbent.

4. Adsorbent according to any one of the preceding claims, the loss on ignition of which, measured at 950°C according to Standard NF EN 196-2 is, in the broad sense, comprised between 4.0 and 7.7%, and preferably between 4.5 and 6.5% and advantageously between 4.8 and 6%, limits included.

5. Adsorbent according to any one of the preceding claims, in the form of agglomerates, the number-average diameter of which is comprised between 0.4 mm and 2 mm, in particular between 0.4 mm and 0.8 mm and preferably between 0.4 mm and 0.65 mm, limits included.

6. Use of an adsorbent according to any one of claims 1 to 5 in the processes of:

  • separation of fractions of aromatic $C_8$ isomers and in particular xylenes,
  • separation of substituted toluene isomers, such as nitrotoluene, diethyltoluene, toluenediamine and others,
  • separation of cresols,
  • separation of polyhydric alcohols.

7. Use according to claim 6, for the separation of para-xylene from fractions of aromatic isomers having 8 carbon atoms.

8. Process for the recovery of para-xylene from fractions of aromatic hydrocarbon isomers comprising 8 carbon atoms, in the liquid phase or in the gas phase, by adsorption of the *para*-xylene by means of an adsorbent according to any one of claims 1 to 5, in the presence of a desorbent.

9. Process for the recovery of para-xylene according to claim 8 of simulated moving bed type, namely of simulated countercurrent type or of simulated cocurrent type.

10. Process for the recovery of para-xylene according to any one of claims 8 and 9, wherein the desorbent is chosen from toluene and para-diethylbenzene.

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 3878127 A
- US 2985589 A
- FR 2925366
- US 3119660 A

- FR 2789914
- US 7820869 B
- US 7812208 B
- FR 2903978
- WO 2008009845 A
- US 5284992 A
- US 5629467 A
- US 4402832 A
- US 4498991 A

**Littérature non-brevet citée dans la description**

- **D.W. BRECK.** Zeolite Molecular Sieves. John Wiley and Sons, 1973, 313
- Principles of Adsorption and Adsorption Processes. Principes de l'Adsorption et des Procédés d'Adsorption. John Wiley & Sons, 1984, 326, , 407
- *Principles of Adsorption and Adsorption Processes,* 248-250

- **LI et al.** Primary Analysis on State of Xylene Adsorption Unit. *Jingxi Shiyou Huagong,* 2004, vol. 4, 54-55
- **D.W. BRECK.** Zeolite Molecular Sieves. John Wiley and Sons, 1973, 314-315
- **RUTHVEN.** Principles of Adsorption and Adsorption Processes. John Wiley & Sons, 1984
- **K. SCHUMANN.** *Microporous and Mesoporous Materials,* 2012, vol. 154, 119-123